(19) Europäisches Patentamt
European Patent Office
Office européen des brevets

(11) Publication number: **0 159 637**
**B1**

(12) # EUROPEAN PATENT SPECIFICATION

(45) Date of publication of patent specification: **07.03.90**

(51) Int. Cl.$^5$: **C 07 D 307/89**

(21) Application number: **85104481.8**

(22) Date of filing: **12.04.85**

(54) **Process for preparing 4-substituted phthalic anhydrides.**

(30) Priority: **16.04.84 US 600248**
**16.04.84 US 600247**

(43) Date of publication of application:
**30.10.85 Bulletin 85/44**

(45) Publication of the grant of the patent:
**07.03.90 Bulletin 90/10**

(84) Designated Contracting States:
**AT BE CH DE FR GB IT LI NL SE**

(56) References cited:
**US-A-2 264 429**
**US-A-2 391 226**
**US-A-4 302 396**

**Journal of American Chemical Society 63, 1542 (1970)**

**Journal of American Chemical Society 72, 3732 (1950)**

(73) Proprietor: **STAUFFER CHEMICAL COMPANY**
**Westport Connecticut 06880 (US)**

(72) Inventor: **Telschow, Jeffrey E.**
**416 Benedict Avenue**
**Tarrytown New York 10591 (US)**

(74) Representative: **Jaeger, Klaus, Dipl.-Chem. Dr. et al**
**Jaeger, Steffens & Köster Patentanwälte**
**Pippinplatz 4a**
**D-8035 München-Gauting (DE)**

(56) References cited:
**Chemical Abstracts, vol.54, no.7, April 10, 1960, Columbus, Ohio, USA. K Alder et al. "Constitution of photochemically prepared esters of norcaradienecarboxylic acid" pages 1960, abstract no. 6577e**

**EP 0 159 637 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

Background of the Invention

1. Field of the Invention

This invention relates to a process for preparing 4-substituted phthalic anhydrides. More particularly, it relates to a process for preparing 4-methylphthalic anhydride.

2. Related Art

Phthalic anhydrides are valuable raw materials for making various useful products. The anhydrides are useful as intermediates in the chemical synthesis of herbicides and particularly in the synthesis of certain herbicides used to protect cereal crops. Other uses for these raw materials include polycyclic dyes, alkyd and epoxy resins, polyesters and plasticizers.

Various processes are known for preparing phthalic anhydrides. In two such processes 4-methyl-1,2,3,6-tetrahydrophthalic anhydride is dehydrogenated either by sulfur or by bromine in acetic acid. Yields of 4-methylphthalic anhydride of 59%—87% are claimed for the former method, while the latter gives only a 16% yield (see Izv. Akad. Nauk SSSR, Ser. Khim. 6, 1315 (1973) English trans. 1271 and D. Craig, J. Am. Chem. Soc. 72, 3732 (1950)). In contrast to the latter, dehydrogenation by bromine of 3-methyl-1,2,3,6-tetrahydrophthalic anhydride gives a 73% yield (M. S. Newman et al., J. Am. Chem. Soc. *63,* 1542 (1941)).

It has now been found that also 4-methyl- and other 4-substituted 1,2,3,6-tetrahydrophthalic anhydrides can be dehydrogenated by bromine in higher yields if the reaction is performed in the presence of an at least catalytic amount of an acid acceptor.

Summary of the Invention

A process for preparing 4-substituted phthalic anhydrides in good yields would be advantageous because of the various useful products that are prepared from these anhydrides. It is an object of the present invention to provide a unique, cost effective process for the preparation of 4-substituted phthalic anhydrides. Other objects and advantages of the present invention are shown throughout the specification.

In accordance with the present invention, it has now been discovered that 4-substituted phthalic anhydrides can be prepared by a process which comprises reacting the correspondingly 4-substituted tetrahydrophthalic anhydride, in particular 1,2,3,6-tetrahydrophthalic anhydride, which is preferably the Diels-Alder addition product of a conjugated diene and maleic anhydride, with bromine in the presence of an acid acceptor. The acid acceptor can be present in catalytic amounts.

Detailed Description of the Invention

The present invention comprises a process for preparing 4-substituted phthalic anhydride of the general formula

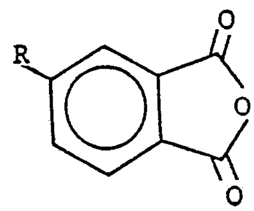

by reacting the corresponding 4-R-tetrahydrophthalic anhydride, in particular 4-R-1,2,3,6-tetrahydrophthalic anhydride, preferably the Diels-Alder addition product of a conjugated diene and maleic anhydride, with bromine in the presence of an acid acceptor.

The substituents R of the 4-substituted phthalic anhydrides of this invention are selected from the group consisting of $C_1$ to $C_{10}$ alkyl, $C_6$ to $C_{14}$ aryl and $C_1$ to $C_{16}$ aralkyl, wherein the alkyl, aryl and aralkyl are optionally substituted with halogens, nitro groups, cyano groups and carboxylic groups.

The process can use the Diels-Alder addition product as a starting material or can comprise a first step of actually preparing this addition product. The Diels-Alder addition product can be derived from other than the Diels-Alder reaction. In addition, the double bond isomers of the Diels-Alder addition product can be used in the process of this invention.

The Diels-Alder addition products used in the process of this invention are formed by reacting maleic anhydride with a correspondingly 2-substituted 1,3-butadiene.

The addition product can be prepared by reacting the maleic anhydride with the 2-R-1,3-butadiene in a nitrogen atmosphere. The maleic anhydride is usually heated until it melts, then the 2-substituted 1,3-butadiene is added slowly under the surface of the melt. When the addition of the diene is completed, the reactants can then be heated up to a reaction temperature of from about 55°C up to about 120°C with temperatures in the upper end of the range from about 100°C up to about 120°C being preferred. The reactants are kept within the reaction temperature range until the reaction is completed, usually for about 1 hour. The reaction can be exothermic, therefore external cooling can be required to maintain the reactants within the reaction temperature range.

After the reaction to form the addition product is completed, excess diene can be stripped from the reaction zone under vacuum at a pressure which will minimize sublimation of the addition product.

The reaction used to prepare the addition product can take place in the presence or absence of an appropriate solvent. However, when the acid acceptor will be added only in catalytic amounts, the absence of a solvent is preferred. An appropriate solvent can be dimethylformamide (DMF). When DMF is used, a solution of the addition product in DMF results and no solids are formed during the reaction. The bromination step can

then be carried out directly upon this DMF/addition product solution after excess diene is stripped from the reaction zone. The DMF in this instance would act both as a solvent and as an acid acceptor reagent.

The stoichiometry of this Diels-Alder addition reaction usually involves one mole of the maleic anhydride reacting with one mole of the conjugated diene to produce one mole of the addition product, therefore it is economically desirable to react equimolar quantities of the reactants. However, a fractional molar excess of the diene is usually used to ensure that all the maleic anhydride is consumed in the reaction.

When the Diels-Alder addition product is used as a starting material and the acid acceptor is present in greater than catalytic amounts, the following procedure can be used in accordance with this invention. The addition product is made up into a solution with a suitable solvent and the acid acceptor reagent is added to this solution. Some examples of suitable solvents are chlorobenzene and DMF. When DMF is used as the solvent, a sufficient quantity is used so that DMF can also participate in the reaction as the acid acceptor reagent.

Bromine can then be added slowly to the reactor. After the bromine addition begins, a hydrogen bromide/acid acceptor adduct will begin to form and may or may not precipitate from solution. The bromine addition can be mildly to moderately exothermic. During the bromine addition, the temperature in the reaction zone is generally maintained at from about 35°C up to about 150°C. When a solvent is used in the reaction a temperature range of from about 35°C up to the boiling point of the solvent is preferred.

After the bromine addition is completed, the temperature within the reaction zone can be slowly increased to a range from about 70°C up to about 180°C to ensure completion of the reaction.

After the reaction is completed, a crude solution remains within the reactor. This crude solution can then be cooled to temperatures from about 0°C up to about 60°C and then water can be added. Two separate layers, an aqueous and an organic layer result, which can be separated. When DMF is used as both the solvent and the acid acceptor reagent, chloroform can be used to extract the phthalic anhydride. Chloroform is then added prior to the addition of water and the aqueous layer can be washed with chloroform after the separation of layers. Chlorobenzene can also be used as an extraction and washing agent. The organic layers can then be combined, concentrated and distilled to form the desired end product, a 4-substituted phthalic anhydride.

When the Diels-Alder addition product is used as a starting material and the acid acceptor is used in a catalytic amount, the following procedure can be used in accordance with this invention. The addition product is heated in a reactor having a nitrogen atmosphere until it melts. The acid acceptor catalyst is first added to the melt, then the temperature is raised to about 120°C. Bromine can then be added slowly to the reactor under the surface of the melt. After the bromine addition begins, hydrogen bromide will slowly begin to evolve, then increase to a fairly constant rate. The bromine addition can be adjusted to such a rate that none of the characteristic bromine color is evident within the liberated hydrogen bromide. The bromine addition can be mildly to moderately exothermic. During the bromine addition, the temperature in the reaction zone is generally maintained at from about 100°C up to about 180°C with from about 135°C up to about 145°C being preferred.

After the bromine addition is completed, the temperature within the reaction zone can be slowly increased up to about 180°C to ensure completion of the reaction. This is evidenced by the cessation of hydrogen bromide evolution. A bubbler containing mineral oil in the exit line can be used to monitor hydrogen bromide evolution.

A crude melt remains within the reactor after the reaction is completed. This melt can be distilled directly, without an aqueous work-up, to produce a high purity 4-substituted phthalic anhydride.

This process can take place in the presence of an appropriate solvent, however the absence of a solvent is preferred. When a solvent is used an aqueous work-up can be required as an additional purification step, especially if the solvent reacts with hydrogen bromide..

The acid acceptors that can be used in this process are varied, however pyridine and dimethylformamide are preferred, with DMF being most preferred. DMF is less costly than pyridine and no solids are formed during the reaction when DMF is used as both the solvent and the acid acceptor reagent.

The stoichiometry of the dehydrogenation reaction usually involves one mole of the addition product reacting with two moles of bromine and four moles of the acid acceptor to produce one mole of the 4-substituted phthalic anhydride. It is therefore economically desirable to react quantities of the reagents that are proportionate to this stoichiometry.

The acid acceptors that can be used in this process as catalysts are varied, however pyridine and dimethylformamide are preferred, with DMF being most preferred. When DMF is the catalyst used, its residue can remain within the reactor with the residue that remains after the distillation of the 4-substituted phthalic anhydride end product, whereas this may not be the case when pyridine is used as the catalyst.

The concentration of the acid acceptor used in this process as a catalyst can range from about 0.1 to 10.0 mass percent with a range from about 1.0 to 5.0 mass percent being preferred. These mass percents are based on the mass of the Diels-Alder addition product used in the reaction.

In a preferred embodiment of this invention, 4-methylphthalic anhydride, (4-MPA), is prepared. This process comprises reacting 4-methyl-1,2,3,6-tetrahydrophthalic anhydride, (4-MTPA), with

bromine in the presence of an acid acceptor. The acid acceptors used can be dimethylformamide, (DMF), or pyridine with DMF being most preferred. This process can be carried out in the presence of an appropriate solvent such as chlorobenzene or even an excess of DMF. The 4-MPA resulting from this process can be distilled after an aqueous work-up/solvent extraction step.

In another preferred embodiment, the acid acceptor is used only in a catalytic amount. In this embodiment, the preferred acid acceptor used can be as described above, however the absence of a solvent is preferred. The 4-MPA resulting from this process can be distilled directly from the reaction zone without the need for an aqueous work-up or other solvent extraction step.

In another preferred embodiment 4-MPA can be prepared by a process which comprises a first step of reacting isoprene and maleic anhydride to form the Diels-Alder addition product, 4-MTPA, then proceeding to react the 4-MTPA with bromine in the presence of an acid acceptor whether in catalytic amount or reagent amount as described above.

The following Examples describe various embodiments of the invention. Other embodiments will be apparent to one of ordinary skill in the art from a consideration of this specification or practice of the invention disclosed herein. It is intended that the specifications and Examples be considered as exemplary only, with the true scope and spirit of the invention being indicated by the Claims which follow the Examples.

Example 1

In a 1 l 3-necked flask fitted with a dropping funnel, a mechanical stirrer, a pot thermometer and a condenser was placed 98.1 g (1.0 mol) of maleic anhydride. The flask was heated in an oil bath until the maleic anhydride melted. Isoprene (69.5 g, 1.02 mol) was then added dropwise to the flask below the surface of the maleic anhydride melt, using an extension tube of PTFE attached to the dropping funnel, at such a rate as to minimize the reflux. The temperature of the reactants in the flask was controlled with intermittent cooling and kept between 55°C and 100°C.

After the addition of the isoprene was completed, the reactor flask was heated to 120°C and maintained at that temperature for 60 min to ensure complete reaction. Excess isoprene was then stripped from the reactor at 13 kPa (100 mm/90°C) for 20 min.

The faintly yellow, molten 4-methyl-1,2,3,6-tetrahydrophthalic anhydride (4-MTPA, mp 58°C—63°C) remained in the reactor flask.

Example 2

A 2 l 3-necked flask was fitted with a heating mantle, a pot thermometer, a 250 ml-dropping funnel, a mechanical stirrer and a condenser. The flask was charged with 98.1 g (1.0 mol) of maleic anhydride and 400 ml of dimethylformamide (DMF), then the resulting solution was heated to 55°C. A total of 110 ml of isoprene (74.9 g, 1.1 mol) was added dropwise to the flask while maintaining the temperature of the reactants within the flask between 55°C to 90°C by means of intermittent cooling in a cold water bath.

After the addition of isoprene was completed, the solution in the reactor flask was heated to 90°C to 100°C range and maintained at that temperature for 60 minutes to ensure complete reaction. The solution was then cooled to 50°C and then excess isoprene was stripped from the reactor at 6.7 kPa (50 mm Hg) for 30 min.

After stripping, the remaining solution was returned to atmospheric pressure, the temperature was adjusted to 60°C, and bromine was added dropwise. The temperature of the solution began to increase with the bromine addition and was allowed to reach a temperature range of between 80°C to 90°C. This temperature range was maintained throughout the 2 h bromine addition by gentle cooling.

After the bromine addition was completed, the dark solution that resulted was heated to a temperature range of between 110°C to 120°C and maintained at that temperature for 1 h. The solution was cooled to below 60°C, diluted with 400 ml of chloroform, and then further cooled to 20°C.

The stirred solution was treated with 400 ml of water and the aqueous phase was separated and extracted again with 400 ml of chloroform. The combined chloroform layers were washed with 600 ml of water and then stripped to a brown crystalline residue via a rotary evaporator.

The crude residue was vacuum distilled using a 7.5 cm-Vigreux column and a short, uncooled condenser to give 132.3 g (82% yield based on maleic anhydride) of a yellow liquid, 4-MPA, which later solidified (bp 150°C—160°C/1.33 kPa (10 mm), mp 75°C—89°C).

Example 3

A solution of 16.6 g (0.10 mol) of 4-MTPA in 60 ml of chlorobenzene and 32.7 ml (32 g, 0.404 mol) of pyridine was prepared in a 3-necked flask. Bromine (10.3 ml, 32.1 g, 0.202 mol) was added dropwise to this solution.

The temperature of the reactants began to increase with the bromine addition and was allowed to reach a temperature range of between 30°C to 42°C. This temperature range was maintained until the bromine addition was completed, then the reactants were heated to 70°C and maintained at this temperature for 30 min.

The reactants were cooled to 25°C and 100 ml of water added to the flask. Two phases formed which were separated, and the aqueous phase was further extracted with two 25 ml portions of chlorobenzene. The combined organic fractions were concentrated under high vacuum to leave 14.1 g (87% yield) of a yellow solid. This yellow solid was virtually pure 4-MPA when analyzed by g.c. and NMR.

Example 4

The following example represents a

generalized procedure.

Molten 4-MTPA (166.1 g, 1.0 mol) was prepared in a 3-necked flask without solvent as described above in Example 1. Dimethylformamide (DMF, 5.0 g, 0.068 mol) was added to the flask.

The content of the flask was then heated to 120°C and stirred mechanically while bromine (103.5 ml, 322.8 g, 2.02 mol) was added dropwise under the surface of the melt. The reactor temperature increased to approximately 140°C and was maintained at between 135°C to 145°C throughout the 2.5 to 3.5 h bromine addition by means of an oil bath thermostatically controlled at 140°C to 145°C. Hydrogen bromide began to evolve a few min after the bromine addition began and was neutralized in a trap containing a sodium hydroxide solution. The bromine addition rate was adjusted so that no bromine color was observed in the hydrogen bromide liberated.

After the addition of the bromine was completed, the dark brown melt was maintained at 140°C for 15 min then slowly heated to 180°C to complete the liberation of hydrogen bromide. After 1 h at 180°C, the crude brown melt remaining was distilled from the flask using a 7.5 cm-Vigreaux column and a short, uncooled condenser. The pale yellow distillate was collected quickly at bp 153°C—157°C/933 Pa (7 mm). After cooling, a pale yellow to white solid product, 4-methylphthalic anhydride (4-MPA), resulted. The 4-MPA had a mass between 124—130 g (76%—80% yield), with a melting point of 78°C—89°C, and an assay by hplc of 95% purity.

Example 5

Molten 4-MTPA (166.1 g, 1.0 mol) was prepared as described in Example 1. Pyridine (8.5 ml, 8.3 g, 0.105 mol) was added to the flask.

The mechanically stirred contents of the flask were maintained at 105°C—120°C as bromine (103.5 ml, 322.8 g, 2.02 mol) was added dropwise under the surface of the melt over 3.5 h. Hydrogen bromide evolved continuously. After the addition was completed, the reactor flask temperature was maintained at 110°C for 2 h. Gc analysis showed that the reaction was incomplete, so another 10 ml of bromine was added at 110°C—120°C over 40 min. The dark brown melt was then heated to 150°—155°C for 4 h to complete the liberation of hydrogen bromide.

The crude melt was distilled under vacuum as in Example 4. After a small forerun, the main fraction was collected at bp 129°C—131°C/200 Pa (7.5 mm). The white solid, 4-MPA, obtained on cooling had a mass of 110.7 g (62% yield) and was 90% pure by hplc assay.

**Claims**

1. A process for preparing a 4-substituted phthalic anhydride according to the general formula

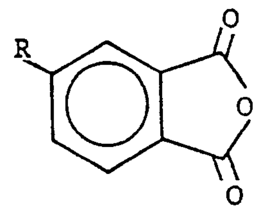

wherein R represents a $C_1$ to $C_{10}$ alkyl, $C_6$ to $C_{14}$ aryl or $C_1$ to $C_{16}$ aralkyl group, and wherein the alkyl, aryl and aralkyl groups are optionally substituted with halogens, nitro groups, cyano groups and carboxylic groups, characterized in that the corresponding 4-R-tetrahydrophthalic anhydride, in particular 4-R-1,2,3,6-tetrahydrophthalic anhydride is reacted with bromine in the presence of an acid acceptor.

2. The process of claim 1, characterized in that a 4-R-1,2,3,6-tetrahydrophthalic anhydride is used wherein R denotes methyl.

3. The process of claim 1 or 2, characterized in that said acid acceptor is dimethylformamide or pyridine.

4. The process of one of the claims 1 to 3, characterized in that a catalytic amount of acid acceptor is used.

5. The process of one of the claims 1 to 4, characterized in that the reaction is carried out in the presence of chlorobenzene as a solvent.

6. The process of claim 4, characterized in that the reaction is carried out in the absence of a solvent.

7. The process of claim 6, characterized in that the phthalic anhydride is distilled directly.

8. A process for preparing a 4-substituted phthalic anhydride as defined in claim 1, characterized by the following steps:

a) reacting a 2-R-1,3-butadiene, wherein R is as defined in claim 1, and maleic anhydride to form a Diels-Alder addition product; and

b) reacting the addition product with bromine in the presence of an acid acceptor.

9. The process of claim 8, characterized in that isoprene is used as the 2-R-1,3-butadiene.

10. The process of claim 8 or 9, characterized in that the acid acceptor is dimethylformamide or pyridine.

11. The process of one of the claims 8 to 10, characterized in that a catalytic amount of acid acceptor is used.

12. The process of one of the claims 8 to 11, characterized in that step b) is carried out in the presence of chlorobenzene as a solvent.

13. The process of claim 8 or 9, characterized in that steps a) and b) are carried out in the presence of a solvent.

14. The process of claim 11, characterized in that steps a) and b) are carried out in the absence of a solvent.

15. The process of claim 14, characterized in that the phthalic anhydride is distilled directly.

**Patentansprüche**

1. Verfahren zur Herstellung eines 4-substituierten Phthalsäureanhydrids der allgemeinen Formel

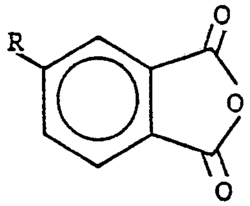

worin R für eine $C_1$ bis $C_{10}$ Alkyl-, $C_6$ bis $C_{14}$ Aryl- oder $C_1$ bis $C_{16}$ Aralkyl-Gruppe steht und worin die Alkyl-, Aryl- und Aralkyl-Gruppen gewünschtenfalls mit Halogenatomen, Nitrogruppen, Cyanogruppen und Carboxylgruppen substituiert sind, dadurch gekennzeichnet, daß das entsprechende 4-R-Tetrahydrophthalsäureanhydrid, insbesondere das 4-R-1,2,3,6-Tetrahydrophthalsäureanhydrid mit Brom in Gegenwart eines Säurefängers umgesetzt wird.

2. Verfahren nach Anspruch 1, dadurch gekennzeichnet, daß ein 4-R-1,2,3,6-Tetrahydrophthalsäureanhydrid verwendet wird, worin R für Methyl steht.

3. Verfahren nach Anspruch 1 oder 2, dadurch gekennzeichnet, daß als Säurefänger Dimethylformamid oder Pyridin verwendet wird.

4. Verfahren nach einem der Ansprüche 1 bis 3, dadurch gekennzeichnet, daß eine katalytische Menge des Säurefängers verwendet wird.

5. Verfahren nach einem der Ansprüche 1 bis 4, dadurch gekennzeichnet, daß die Reaktion in Gegenwart von Chlorbenzol als Lösungsmittel durchgeführt wird.

6. Verfahren nach Anspruch 4, dadurch gekennzeichnet, daß die Reaktion in Abwesenheit eines Lösungsmittels durchgeführt wird.

7. Verfahren nach Anspruch 6, dadurch gekennzeichnet, daß das Phthalsäureanhydrid direkt destilliert wird.

8. Verfahren zur Herstellung eines 4-substituierten Phthalsäureanhydrids wie in Anspruch 1 definiert, gekennzeichnet durch die folgenden Schritte:

(a) Umsetzen eines 2-R-1,3-Butadiens, worin R die in Anspruch 1 angegebenen Bedeutungen besitzt, mit Maleinsäureanhydrid zur Bildung eines Diels-Alder-Additionsproduktes und

(b) Umsetzen des Additionsproduktes mit Brom in Gegenwart eines Säurefängers.

9. Verfahren nach Anspruch 8, dadurch gekennzeichnet, daß Isopren als 2-R-1,3-Butadien verwendet wird.

10. Verfahren nach Anspruch 8 oder 9, dadurch gekennzeichnet, daß Dimethylformamid oder Pyridin als Säurefänger verwendet wird.

11. Verfahren nach einem der Ansprüche 8 bis 10, dadurch gekennzeichnet, daß der Säurefänger in katalytischer Menge eingesetzt wird.

12. Verfahren nach einem der Ansprüche 8 bis 11, dadurch gekennzeichnet, daß Schritt (b) in Gegenwart von Chlorbenzol als Lösungsmittel durchgeführt wird.

13. Verfahren nach Anspruch 8 oder 9, dadurch gekennzeichnet, daß die Schritte (a) und (b) in Gegenwart eines Lösungsmittels durchgeführt werden.

14. Verfahren nach Anspruch 11, dadurch gekennzeichnet, daß die Schritte (a) und (b) in Abwesenheit eines Lösungsmittels durchgeführt werden.

15. Verfahren nach Anspruch 14, dadurch gekennzeichnet, daß das Phthalsäureanhydrid direkt destilliert wird.

**Revendications**

1. Procédé pour la préparation d'un anhydride phtalique substitué en position 4 selon la formule générale suivante

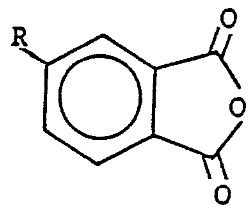

dans laquelle R représente un radical alkyle en $C_1$ à $C_{10}$, aryle en $C_6$ à $C_{14}$ ou aralkyle en $C_1$ à $C_{16}$, et où les radicaux alkyle, aryle et aralkyle sont facultativement substitués par des halogènes, des groupes nitro, des groupes cyano et des groupes carboxyliques, caractérisé en ce que l'anhydride 4-R-tétrahydrophtalique correspondant, en particulier l'anhydride 4-R-1,2,3,6-tétrahydrophtalique, est mis à réagir avec du brome en présence d'un accepteur d'acide.

2. Procédé selon la revendication 1, caractérisé en ce qu'on utilise un anhydride 4-R-1,2,3,6-tétrahydrophtalique dans lequel R désigne le radical méthyle.

3. Procédé selon les revendications 1 ou 2, caractérisé en ce que ledit accepteur d'acide est le diméthylformamide ou la pyridine.

4. Procédé selon l'une des revendications 1 à 3, caractérisé en ce qu'on utilise une quantité catalytique d'accepteur d'acide.

5. Procédé selon l'une des revendications 1 à 4, caractérisé en ce que la réaction est mise en oeuvre en présence de chlorobenzène servant de solvant.

6. Procédé selon la revendication 4, caractérisé en ce que la réaction est mise en oeuvre en l'absence de solvant.

7. Procédé selon la revendication 6, caractérisé en ce que l'anhydride phtalique est directement distillé.

8. Procédé pour la préparation d'un anhydride phtalique substitué en position 4 selon la revendication 1, caractérisé en ce qu'il comprend les étapes consistant

a) à faire réagir un 2-R-1,3-butadiène, où R est un tel que défini dans la revendication 1, et de l'anhydride maléique pour former un produit

d'addition de Diels-Alder; et

b) à faire réagir le produit d'addition sur du brome en présence d'un accepteur d'acide.

9. Procédé selon la revendication 8, caractérisé en ce que l'isoprène utilisé est le 2-R-1,3-butadiène.

10. Procédé selon les revendications 8 ou 9, caractérisé en ce que l'accepteur d'acide est le diméthylformamide ou la pyridine.

11. Procédé selon l'une des revendications 8 à 10, caractérisé en ce qu'on utilise une quantité catalytique de l'accepteur d'acide.

12. Procédé selon l'une des revendications 8 à 11, caractérisé en ce que l'étape b) est mise en oeuvre en présence de chlorobenzène servant de solvant.

13. Procédé selon les revendications 8 ou 9, caractérisé en ce que les étapes a) et b) sont mises en oeuvre en présence d'un solvant.

14. Procédé selon la revendication 11, caractérisé en ce que les étapes a) et b) sont mises en oeuvre en l'absence de solvant.

15. Procédé selon la revendication 14, caractérisé en ce que l'anhydride phtalique est directement distillé.